(19) Europäisches Patentamt
European Patent Office
Office européen des brevets

(11) Veröffentlichungsnummer: **0 240 854**
**A1**

# (12) EUROPÄISCHE PATENTANMELDUNG

(21) Anmeldenummer: **87104465.7**

(22) Anmeldetag: **26.03.87**

(51) Int. Cl.⁴: **C07D 251/70**, C07D 403/04, A61K 31/53

Patentansprüche für folgenden Vertragsstaten: AT + ES + GR.

(30) Priorität: **05.04.86 DE 3611427**

(43) Veröffentlichungstag der Anmeldung: **14.10.87 Patentblatt 87/42**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Kreutzberger, Alfred, Prof. Dr.**
**Wormser Strasse 171f,**
**D-6500 Mainz(DE)**
Erfinder: **Söder, Alfons, Dr.**
**Kauber Weg 1**
**D-6000 Frankfurt am Main(DE)**
Erfinder: **Gerhards, Hermann Josef, Dr.**
**Wacholderweg 4,**
**D-6238 Hofheim am Taunus(DE)**

## (54) Bis-tertiärbutylamino-substituierte 1,3,5-Triazinderivate, Verfahren zu ihrer Herstellung, diese Verbindungen enthaltende Arzneimittel und ihre Verwendung.

(57) 2,4-Bis-(tertiärbutylamino)-1,3,5-triazine der Formel I

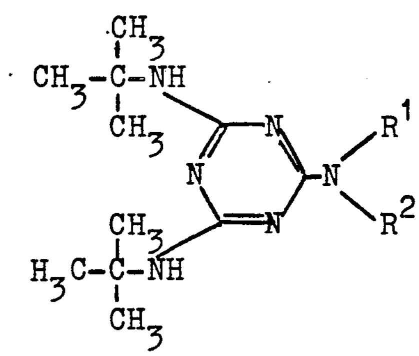

I

worin $R^1$ und $R^2$ die angegebenen Bedeutungen haben. Verfahren zur Herstellung dieser Verbindungen und Arzneimittel, die diese Triazinderivate enthalten, werden beschrieben. Die Verbindungen haben anticonvulsive und anxiolytische Wirkungen.

EP 0 240 854 A1

# Bis-tertiärbutylamino-substituierte 1,3,5-Triazinderivate, Verfahren zu ihrer Herstellung, diese Verbindungen enthaltende Arzneimittel und ihre Verwendung.

Die Erfindung betrifft neue bis-tertiärbutylamino-substituierte 1,3,5-Triazinderivate, Verfahren zur Herstellung dieser Verbindungen, pharmazeutische Mittel, welche die erfindungsgemäßen aktiven Verbindungen enthalten und deren Verwendung als Arzneimittel, insbesondere zur Prophylaxe und Behandlung der Epilepsie und von Angstzuständen.

Das 2,4,6-Tris-tertiärbutylamino-1,3,5-triazin wurde bereits beschrieben (vgl. z.B. US-Patent 2.691.021). Entsprechend den Angaben in der Literatur findet dieses 1,3,5-Triazinderivat Verwendung bei der Herstellung von synthetischen Harzen und oberflächenaktiven Stoffen (US-Patent 2.691.021). Die Verwendung gegen Mycobakterien (US-Patent 3.591.693) und gegen Fortschreiten von Arthritis (US-Patent 4.269.832) wurde ebenfalls beschrieben.

Überraschenderweise wurde nun gefunden, daß 2,4-Bis-(tertiärbutylamino)-1,3,5-triazine der allgemeinen Formel I

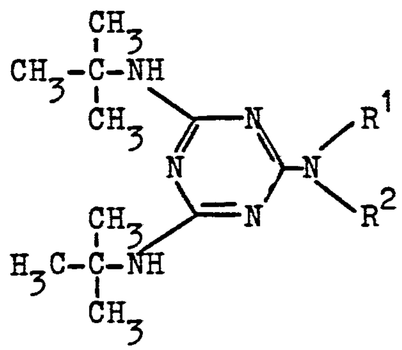

I

antiepiletische und anxiolytische Wirkungen aufweisen.

Die Erfindung betrifft daher 2,4-Bis-(tertiärbutylamino)-1,3,5-triazine der allgemeinen Formel I, worin $R^1$ $C_1$-$C_6$-Alkyl, $C_2$-$C_6$-Monohydroxyalkyl, $C_3$-$C_6$-Dihydroxyalkyl, $C_4$-$C_6$-Trihydroxyalkyl, Alkoxyalkyl mit insgesamt 3 bis 7 Kohlenstoffatomen, $C_3$-$C_7$-Alkenyl, $C_3$-$C_7$-Cycloalkyl, Cycloalkylalkyl mit insgesamt 4 bis 7 Kohlenstoffatomen, $C_1$-$C_6$-Alkylamino, Dialkylamino mit insgesamt 2 bis 10 Kohlenstoffatomen, oder einen Amino

rest der Formel 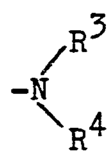 ,

worin

$R^3$ und $R^4$ zusammen eine Alkylenkette mit 3 bis 6 Methylengruppen darstellen, von denen gegebenenfalls eine durch O oder S ersetzt ist oder von denen eine gegebenenfalls mit $C_1$-$C_3$-Alkyl substituiert ist, bedeutet und

$R^2$ Wasserstoff, $C_1$-$C_6$-Alkyl, $C_2$-$C_6$-Hydroxyalkyl, Alkoxyalkyl mit insgesamt 3 bis 7 Kohlenstoffatomen oder $C_3$-$C_7$-Alkenyl ist oder

$R^1$ und $R^2$ bilden gemeinsam mit dem Stickstoffatom ein 4 bis 7 gliedriges, 1, 2 oder 3 Stickstoffatome enthaltendes, ungesättigtes, teilhydriertes oder gesättigtes heterocyclisches Ringsystem, das gegebenenfalls mit $C_1$-$C_3$-Alkyl substituiert ist, oder $R^1$ und $R^2$ bilden gemeinsam mit dem Stickstoffatom den Morpholin oder Thiomorpholinrest,

wobei jedoch nicht gleichzeitig $R^1$ Tertiärbutyl und $R^2$ Wasserstoff bedeuten.

In den vorstehenden und folgenden Ausführungen werden unter Alkyl, Alkoxy und Alkenyl sowohl geradkettige als auch verzweigte Reste verstanden. Die Alkoxyalkylreste weisen 1 oder 2 Alkoxygruppen auf.

$R^1$ bedeutet vorzugsweise $C_1$-$C_6$-Alkyl, $C_4$-$C_6$-Mono-, Di-oder Tri-hydroxyalkyl, $C_5$-$C_6$-Cycloalkyl, Dialkylamino mit insgesamt 2 bis 4 Kohlenstoffatomen, oder einen

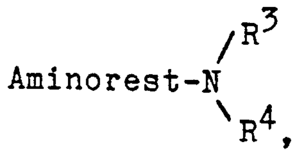 worin $R^3$, $R^4$ und das Stickstoffatom gemeinsam einen Pyrrolidino-, Piperidino, Morpholino-oder Thiomorpholinorest darstellen.

$R^2$ ist vorzugsweise Wasserstoff, $C_1$-$C_6$-Alkyl oder $C_4$-$C_6$-Monohydroxyalkyl.

Falls $R^1$ und $R^2$ gemeinsam mit dem Stickstoffatom einen heterocyclischen Ring bilden, so ist dieser vorzugsweise 5-6-gliedrig, weist 1 bis 2 Stickstoffatome auf und ist gesättigt oder ungesättigt.

Die Erfindung betrifft ferner Verfahren zur Herstellung der Verbindungen der allgemeinen Formel I, welche dadurch gekennzeichnet sind, daß man in an sich bekannter Weise

a) eine Verbindung der allgemeinen Formel II

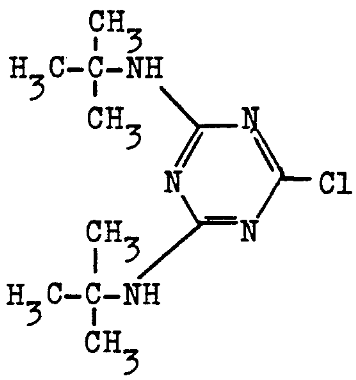

II

und ein Amin der allgemeinen Formel III,

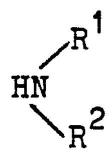

worin $R^1$ und $R^2$ die gleichen Bedeutungen wie in der Formel I haben, miteinander umsetzt oder

b) eine Verbindung der allgemeinen Formel IV,

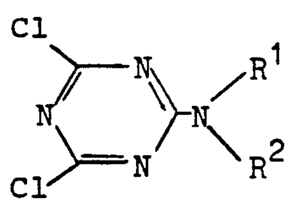

worin $R^1$ und $R^2$ ebenfalls die gleichen Bedeutungen wie in der Formel I haben, mit tertiär Butylamin umsetzt.

Die Herstellung der erfindungsgemäßen Verbindungen gemäß Formel I nach Verfahren a aus einer Verbindung der allgemeinen Formel II und einem Amin der allgemeinen Formel III wird zweckmäßigerweise bei einer Temperatur zwischen 20° und 200°C, vorteilhaft in Gegenwart eines organischen Lösungsmittels, gegebenenfalls in einem Druckgefäß, durchgeführt. Geeignete inerte Lösungsmittel sind hierfür aliphatische, alicyclische und aromatische Kohlenwasserstoffe, insbesondere Petrolether, Cyclohexan, Chlorbenzol, Toluol, Xylol oder Mesitylen, Ether wie Tetrahydrofuran, Dioxan, oder Dimethoxyethan sowie Sulfolan oder α-Pyridon. Bevorzugte Lösungsmittel sind Cyclohexan, Tetrahydrofuran, Toluol und Xylol.

Die Herstellung der erfindungsgemäßen Verbindungen gemäß Formel I nach Verfahren b aus einer Verbindung als allgemeinen Formel IV mit tertiär Butylamin erfolgt vorzugsweise bei einer Temperatur zwischen 80° und 200°C, insbesondere bei einer Temperatur zwischen 100° und 170°C im Druckgefäß in Gegenwart eines der oben aufgeführten Lösungsmittels.

Im Verfahren a und b kann das Lösungsmittel auch durch einen Überschuß des Amins ersetzt werden. Nach beendeter Umsetzung wird das gewünschte Reaktionsprodukt vom gebildeten Aminhydrochlorid und dem Lösungsmittel abgetrennt. Die Reinigung erfolgt vorteilhaft mittels Umkristallisation oder Säulenchromatographie. Als Amine der Formel III können außer den in den Beispielen genannten vorzugsweise die folgenden Amine eingesetzt werden:

Primäre Amine: Methylamin, Ethylamin, 1-Propylamin, 1-Butylamin, Isobutylamin, 2-Pentylamin, Isopentylamin, Neopentylamin, 1-Hexylamin, 2-Hexylamin, 3-Hexylamin, 1-Methylpentylamin, 2-Ethylbutylamin, 2-Hydroxyethylamin, 2-Hydroxypropylamin, 3-Hydroxypropylamin, 2-Amino-1-butanol, 4-Amino-1-butanol, 2-Methoxyethylamin, 2-(Diethoxy)-ethylamin, Allylamin, Cyclopropylamin, Cyclobutylamin und Cyclohexylmethylamin.

Sekundäre Amine: Dimethylamin, Dipropylamin, Dibutylamin, Methylbutylamin, Methylisopropylamin, Methyl-tert. butylamin, Diallylamin, Methylallylamin, Cyclohexylmethylamin, Cyclohexylallylamin.

Hydrazine: Methylhydrazin, Ethylhydrazin, Propylhydrazin, Isopropylhydrazin, Butylhydrazin, 2-Butylhydrazin, Isobutylhydrazin, tert. Butylhydrazin, N,N-Diethylhydrazin, N,N'-Diethylhydrazin, N,N Methylpropylhydrazin, N-Methyl-N'-butylhydrazin, N-Aminopyrrolidin, N-Aminothiomorpholin.

Cyclische Amine: 3-Pyrrolin, 4-Methylpiperidin, 2-Ethylpiperidin, 3,5-Dimethylpiperidin, 1,2,5,6-Tetrahydropyridin, Hexamethylenimin, Morpholin, Thiomorpholin, Imidazol, 2-Methylimidazol, 4-Ethylimidazol, Pyrazol und 1,2,4-Triazol.

Die Verbindungen der allgemeinen Formel I besitzen pharmakologische Eigenschaften, die sie als mögliche Krampfverhindernde und Angst-lösende Pharmaka charakterisieren.

Die Krampf-verhindernde (antikonvulsive) Wirkung der erfindungsgemäßen Verbindungen wurde an Mäusen gegen Pentetrazol-und gegen Elektroschock-induzierte Krämpfe in der Anordnung von SWINYARD geprüft.

1. Antagonismus gegen Pentetrazol-induzierte Krämpfe (Maus)

Methode (E.A.SWINYARD, J.Pharmacol.Exp.Ther. 106, (1952), 319-330):

Gruppen von 10 männlichen Mäusen (Stamm: NMRI, SPF-71, KF) im Gewicht von 18-22 g werden in diesem Versuch verwendet. Die zu prüfenden Substanzen werden in einer 1 %-igen Aufschwemmung von ®Tylose (Methyl-hydroxy-ethyl-cellulose) in Wasser suspendiert und per Schlundsonde in einem Volumen von 10 ml/kg Körpergewicht oral verabreicht. Die Kontrollgruppe erhält ein entsprechendes Volumen Tylosesuspension ohne Prüfsubstanz. Pentetrazol (Cardiazol®) wird in wässriger Lösung in einer Dosis von 125 mg/kg Körpergewicht 1 Stunde nach Verabreichung der Prüfsubstanzen subcutan injiziert. Diese Dosis erzeugt bei 90-100 % der Kontrolltiere innerhalb der Beobachtungszeit von 60 Minuten nach der Injektion tonische Extensorkrämpfe der hinteren Extremitäten. Als Schutzwirkung der Prüfsubstanz wird gewertet, wenn die Anzahl der krampfenden Tiere gegenüber der Kontrollgruppe vermindert ist. Bei vorhandener Schutzwirkung der Prüfsubstanz wird eine mittlere effektive Dosis (ED-50) grafisch (Litchfield and Wilcoxon) oder rechnerisch (Probitanalyse) ermittelt.

2. Antagonismus gegen Elektroschock-induzierte Krämpfe (Maus).

Methode (E.A.SWINYARD, In: Experimental Models of Epilepsy, Raven Press, New York, 1972, p. 433-458):

Gruppen von 6 männlichen Mäusen (Stamm: NMRI, SPF-71, KF) im Gewicht von 18-22 g werden in diesem Versuch verwendet. Die zu prüfenden Substanzen werden in einer 1 %igen Aufschwemmung von Tylose (Methyl-hydroxy-ethyl-cellulose) in Wasser suspendiert und per Schlundsonde in einem Volumen von 10 ml/kg Körpergewicht oral verabreicht. Die Kontrollgruppe erhält ein entsprechendes Volumen Tylosesuspension ohne Prüfsubstanz. Eine Stunde nach Prüfsubstanzgabe wird den Tieren über Cornea-Elektroden ein Elektroschock verabreicht (Stromstärke 12-22 mA, Dauer 0,2 sec, 50 Hz), nachdem vorher an einer zweiten Kontrollgruppe die Stromstärke ermittelt worden war, die bei 100 % der Tiere eine tonischen Extensorkrampf der hinteren Extremitäten hervorruft. Die Anzahl der durch die Prüfsubstanz vor einem Extensorkrampf geschützten Tiere dient als Mass der antikonvulsiven Wirkung in diesem Test. Bei vorhandener Schutzwirkung der Prüfsubstanz wird eine mittlere effektive Dosis (ED-50) grafisch (Litchfield and Wilcoxon) oder rechnerisch (Probitanalyse) ermittelt.

Die ermittelten 50 %-Hemmdosen bei oraler Gabe liegen zwischen 2.0 und 30 mg/kg beim Pentetrazol-Krampf und 14 und 46 mg/kg bei Elektroschockinduzierten Krämpfen (siehe Tabelle).

Die Angst-lösende (anxiolytische Wirkung) der erfindungsgemäßen Verbindungen wurde im "Lick-shock-conflict-Test" nach VOGEl und im "Geller-Seifter-Konflikt-Test" nach GELLER und SEIFTER an WISTAR-Ratten geprüft.

3. Lick-shock-conflict-Test

Methode (VOGEL, J.R., Psychopharmacologia 21, (1971), 1-7

Männliche Wistar Ratten aus eigener Aufzucht (SPF Hattersheim) im Gewicht zwischen 90 und 120 g werden verwendet. Den Tieren wird für 48 Stunden vor Testbeginn das Trinkwasser entzogen. Zum Test werden die Tiere in eine Plastikbox (14x12x28 cm, BxTxH) gesetzt, die mit einer Wasserflasche mit Metall-Trinkrohr ausgestattet ist und die außerdem erlaubt, über eine elektronische Schaltung die Anzahl der Kontakte der Zunge des Tieres mit dem Trinkrohr zu messen. Der Boden der Box besteht aus Metallstäben, die durch die Steuerungselektronik unter Strom gesetzt werden können. Die Tiere haben nach dem Einsetzen in die Box 5 min. Zeit, um das Trinkrohr zu finden und 50-mal daran zu lekken. Tiere, die das Trinkrohr innerhalb dieser Zeit nicht gefunden haben, werden für den Versuch nicht verwendet. Nach diesen 50 Leckvorgängen (lickings) werden Trinkrohr und Bodenstäbe für jeweils 5 sec unter Strom gesetzt (Gleichstrom 300 µA) und dann für weitere 5 sec. wieder freigegeben. Diese Folge wird alternierend für eine Zeit von 5 min. weitergeführt, wobei die Zahl der Trinkrohr-Kontakte des Tieres während der Reizstrom-und der reizstromfreien Phase auf verschiedenen elektronischen Zählern registriert werden.

Gruppen von jeweils acht Tieren pro Dosis werden mit den Prüfsubstanzen, die in einem 1 %igen Tylose-Gel aufgeschwemmt sind, oral per Schlundsonde behandelt. Das Injektionsvolumen beträgt 5 ml/kg Körpergewicht. Die Prüfung in der obg. Testapparatur erfolgt 1 und 2 Std. nach Applikation der Prüfsubstanzen. Die Anzahl der Trinkrohr-Kontakte in der Reizstrom-Phase dient als Prüf-Variable. Die mittlere Anzahl der Kontakte in dieser Phase bei der Kontrollgruppe wird zu 100 % gesetzt und Zu-oder Abnahme der Kontaktzahl bei den mit Prüfsubstanz behandelten Tieren wird prozentual in Bezug auf die Kontrollgruppe ausgedrückt.

Anxiolytika bewirken in diesem Test gewöhnlich eine deutliche Zunahme der Wasseraufnahme (lickings) in der Reizstrom-Phase gegenüber den unbehandelten Kontrollen. Falls eine lineare oder logarithmische Beziehung zwischen Dosis und Wirkung gegeben ist, wird eine ED + 100 (d.i. die Dosis, die eine Zunahme der Wasseraufnahme um 100 % gegenüber der Kontrollgruppe bewirkt) mittels Regressionsanalyse errechnet. Ist eine lineare Dosisabhängigkeit nicht gegeben, so wird eine minimal effektive Dosis (MED) bestimmt, d.i. die niedrigste Dosis der Prüfsubstanz, die noch eine statistisch signifikante Zunahme der Wasseraufnahme im Vergleich zur Kontrollgruppe bewirkt ($p = 0,05$, DUNNET-Test).

4. Geller-Seifter-Konflikt-Test

Methode (Geller, I.und Seifter, J., Psychopharmacologia 1 (1962), 482):

Männliche Wistar Ratten aus eigener Aufzucht (SPF Hattersheim) im Gewicht zwischen 240 und 370 g werden verwendet und Versuchsgruppen zu je 8 Tieren zugeordnet. Je 4 Tiere werden in Plastikkäfigen (56x38x20 cm) zusammengesetzt und durch abgewogene Futtermengen auf ca. 80 % ihres normalen Körpergewichts gehalten.

Die Tiere werden trainiert, in einer SKINNER-Box eine Taste zu drücken, um eine Belohnung in Form von gesüßter Kondensmilch zu erhalten. Die Box enthält zwei Tasten mit Mikroschaltern, einen Lautsprecher, ein Hauslicht, zwei Signallichter über den Tasten sowie einen Fußboden aus Metallstäben. Das Trainingsschema wurde der Arbeit von GELLER und SEIFTER (1962) in der Modifikation von DAVIDSON & COOK (Psychopharmacologia 15 (1969), 159-168) entlehnt:
Jede Sitzung besteht aus vier 15-Minuten-Abschnitten, die alle aus einer 12-minütigen "Variable Interval" (VI) Phase und einer 3-minütigen "Fixed ration" (FR) Phase zusammengesetzt sind. Während der VI-Phase

erhalten die Tiere auf Tastendruck Milchbelohnungen in einem durch einen Zufallsgenerator gesteuerten Intervall von 10-110 sec. mit einem Mittelwert um 60 15 sec. Während der FR-Phase erhalten die Tiere für jeden Tastendruck eine Belohnung, zusätzlich wird jedoch bei jedem 3. Tastendruck ein schmerzhafter elektrischer Reiz über die Fussbodenstäbe verabreicht, um eine Konfliktsituation zu erzeugen. Die Stromstärke des elektrischen Reizes wird für jedes Tier individuell so eingestellt (0,3-0,6 mA), daß die Tastendruckrate in der gesamten FR-Phase zwischen 5 und 15 liegt.

Das Training findet an 5 Tagen in der Woche statt, Versuche mit Prüfsubstanzen werden einmal wöchentlich durchgeführt. Da die Tiere als ihre eigene Kontrolle dienen, werden vor jedem Versuch mit Prüfsubstanzen mindestens zwei Vorwerte ohne Prüfsubstanz durchgeführt. Die zu prüfenden Verbindungen werden in einem 1 %igen Tylose-Gel suspendiert und 30 min vor Testbeginn oral per Schlundsonde in einem Volumen von 2 ml/kg verabreicht. Veränderungen der Tastendruckrate in der VI-Phase werden als Beeinflussung der motorischen Aktivität und Steigerung der Tastendruckrate in der FR-Phase werden als Anzeichnen für eine "Antikonflikt-" bzw. "anxiolytische" Wirkung gewertet. Bestimmt wird meist die minimal effektive Dosis (MED) der Prüfsubstanz, d.i. die niedrigste geprüfte Dosis, die noch eine statistisch signifikante Änderung der Tastendruckrate bewirkt ($p = 0,05$; WILCOXON matched pairs signed rank test).

Die in diesen beiden Versuchsmodellen ermittelten MED's nach oralen Gaben liegen zwischen 2,5 und 30 mg/kg Versuchstier (s. Tabelle). In der Tabelle sind außerdem die prozentualen Zunahmen der angenommenen Belohnungen (Wasser bzw. Milch) angegeben.

Aus der nachfolgenden Tabelle lassen sich die Ergebnisse der vorstehend beschriebenen Tierversuche entnehmen. Sie zeigen die antiepileptische und anxiolytische Wirkung der erfindungsgemäßen bistertiärbutylaminosubstituierten 1,3,5-Triazin-Derivate.

| Verbindung | Ptz | ECS | Lick-Sh. | Geller |
|---|---|---|---|---|
| gem. | Maus | Maus | Ratte | Ratte |
| Beispiel | ED 50 in mg/kg p.o. | | MED in mg/kg p.o. | |
| 1 | 4.2 | 32 | 10:+ 63 % | 3:+ 91 % |
| | 2.1 | | | 10:+ 97 % |
| | | | | 30:+382 % |
| 2 | 2.4 | 30 | 30:+ 74 % | - |
| | | | 10:+ 61 % | - |
| 3 | 3.2 | 30 | 30:+ 87 % | - |
| | | | 10:+ 75 % | - |
| 4 | 8.3 | 30 | 10:+ 82 % | 2,5:+22 % |
| 5 | 12.2 | 30 | 30:+ 34 % | - |
| 6 | 10.0 | 20.7 | 10:+103 % | 10:+ 61 % |
| | | | | 10:+222 % |
| 7 | 6.9 | 14.5 | 30:+101 % | - |
| 8 | 2.3 | 46.6 | 10:+154 % | 10:+ 76 % |
| | | | 30:+230 % | 30:+ 91 % |
| 9 | 10.3 | 24.7 | 30:+ 68 % | - |
| 10 | 30 | 19.7 | 30:+ 61 % | |

Da epileptische Anfälle häufig durch Streß und Angstsituationen ausgelöst werden, ist die Kombination von antiepileptischer und anxiolytischer Wirkung der erfindungsgemäßen Verbindungen für die Therapie epileptischer Erkrankungen besonders wertvoll.

Die Erfindung betrifft ferner die Anwendung von 2,4-Bis-(tertiärbutylamino)-1,3,5-triazinderivaten der Formel I bei der Behandlung und Prophylaxe epileptischer Erkrankungen und von Angstzuständen, weiterhin die Verwendung der genannten Verbindung bei der Herstellung von Arzneimitteln, die zur Behandlung und Prophylaxe der vorstehend genannten Krankheiten eingesetzt werden.

Ein weiterer Gegenstand der Erfindung sind Arzneimittel, die diese Verbindung enthalten.

Die Arzneimittel werden nach an sich bekannten, dem Fachmann geläufigen Verfahren hergestellt. Als Arzneimittel wird eine pharmakologisch wirksame Menge einer erfindungsgemäßen Verbindung als Wirkstoff entweder als solche, oder vorzugsweise in Kombination mit geeigneten Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorzugsweise zwischen 10 und 75 % beträgt.

Welche Hilfsstoffe für die gewünschte Arzneimittelformulierung geeignet sind, ist dem Fachmann aufgrund seines Fachwissens geläufig. Neben Tablettenhilfsstoffen, Lösemitteln, Gelbildnern, Suppositorengrundlagen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Der Wirkstoff kann oral, parenteral, intravenös oder rektal appliziert werden, wobei die orale Applikation bevorzugt ist.

Für eine orale Anwendungsform wird die aktive Verbindung mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können zum Beispiel Gummi arabicum, Magnesia, Magesiumcarbonat, Milchzucker, Glucose oder Stärke, insbesondere Maisstärke verwendet werden. Dabei kann die Zubereitung sowohl als Trocken-als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation wird der Wirkstoff gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen zum Beispiel in Frage Wasser, physiologische Kochsalzlösung oder Alkohole, zum Beispiel Ethanol, Propanol, Glycerin, daneben auch Zuckerlösungen wie Glucose-oder Mannitlösungen, oder auch eine Mischung aus den verschiedenen genannten Lösungsmitteln.

Als täglich zu verabreichende Dosis ist eine solche zu wählen, die der Wirksamkeit des verwendeten Triazinderivates und dem gewünschten Effekt angepaßt ist. Pro Patient werden täglich etwa 5 bis 200 mg, vorzugsweise oral, verabreicht. Bei der Anwendung als Antiepileptikum beträgt die Dosierung vorzugsweise 10 bis 100 mg täglich, wobei die Dosierung je Einheit zweckmäßigerweise 2,5 bis 25 mg beträgt. Als Anxiolytikum werden täglich etwa 5 bis 50 mg in Einzeldosierungen zwischen etwa 1,0 und 20 mg verabreicht. Es können selbstverständlich auch darüber oder darunter liegende Dosierungseinheiten verwendet werden, die gegebenenfalls vor Applikation zu teilen bzw. zu vervielfachen sind.

Beispiel 1

<u>2,4-Bis-(tert.butylamino)-6-piperidino-1,3,5-triazin</u>

25,8 g 2,4-Bis-(tert.butylamino)-6-chlor-1,3,5-triazin werden in einem Gemisch von 18 g Piperidin und 300 ml Tetrahydrofuran unter Rühren gelöst. Dann erhitzt man das Reaktionsgemisch am Rückflußkühler zum Sieden bis zur völligen Umsetzung, die dünnschichtchromatographisch mit Hilfe von Merck DC-Fertigplatten Kieselgel 60F-254, Detektion unter der UV-Lampe 254 nm, Fließmittel Chloroform/Methanol, 9:1 (v/v) $R_f$0,81, verfolgt werden kann. In einem Rotationsverdampfer zieht man das Lösungsmittel im Vakuum ab und nimmt den Rückstand im Zweiphasensystem (250 ml Methylenchlorid/250 ml Wasser) auf. Die Methylenchloridlösung gibt man nach der Abtrennung der wäßrigen Phase zur Reinigung über eine Aluminiumoxid/neutral-Säule. Aus der farblosen Lösung erhält man das 2,4-Bis-(tert.butylamino)-6-piperidino-1,3,5-triazin in kristalliner Form vom Schmelzpunkt 208°C.

Ausbeute: 28,8 g (94 % der Theorie)

Gemäß Beispiel 1 erhält man unter Verwendung der oben angeführten Lösungsmittel weitere erfindungsgemäße Stoffe (Beispiele 2 bis 11):

Beispiel 2

2,4-Bis-(tert.butylamino)-6-pyrrolidino-1,3,5-triazin Fp. 246°C

Beispiel 3

2,4-Bis-(tert.butylamino)-6-(piperidino-amino)-1,3,5-triazin Fp. 206°C

Beispiel 4

2,4-Bis-(tert.butylamino)-6-(morpholino-amino)-1,3,5-triazin Fp. 185°C

Beispiel 5

2,4-Bis-(tert.butylamino)-6-imidazolyl-1,3,5-triazin Fp. 187°C

Beispiel 6

2,4-Bis-(tert.butylamino)-6-(N'-dimethylhydrazino)-1,3,5-triazin Fp. 193°C

Beispiel 7

2,4-Bis-(tert.butylamino)-6-diethylamino-1,3,5-triazin Fp. 121°C

Beispiel 8

2,4-Bis-(tert.butylamino)-6-isopropylamino-1,3,5-triazin Fp. 146°C

Beispiel 9

2,4-Bis-(tert.butylamino)-6-cyclopentylamino-1,3,5-triazin Fp. 150°C

Beispiel 10

2,4-Bis-(tert.-butylamino)-6-(1,2-dimethylpropylamino)-1,3,5-triazin Fp. 143°C

Beispiel 11

2,4-Bis-(tert.butylamino)-6-(4-hydroxybutylamino)-1,3,5-triazin Fp. 175°C

Beispiel 12

2,4-Bis-(tert.butylamino)-6-(1,1-dimethyl-3-hydroxypropylamino)-1,3,5-triazin

12,9 g 2,4-Bis-(tert.butylamino)-6-chlor-1,3,5-triazin und 13,5 g 3-Methyl-3-amino-butanol (1) werden mit 300 ml p-Xylol im Druckgefäß 24 Stunden auf 150°C gehalten. Nach dem Erkalten rührt man das Reaktionsgemisch mit 300 ml Wasser aus. Der Rückstand der organischen Phase wird nun mit 200 ml Petroläther ausgerührt. Das gewünschte Reaktionsprodukt zeigt nach Isolierung und Trockung im Dünnschichtchromatogramm (Bedingungen wie in Beispiel 1 angeführt) $R_f$ = 0,43. Schmelzpunkt 147°C / Ausbeute 15,3 g (94,2 % der Theorie).

Gemäß Beispiel 12 wurden die folgenden erfindungsgemäßen Stoffe erhalten:

Beispiel 13

2,4-Bis-(tert.butylamino)-6-N-methyl-N-butylamino-1,3,5-triazin
Fp. 139°C

Beispiel 14

2,4-Bis-(tert.butylamino)-6-(2-butylamino)-1,3,5-triazin Fp. 133°C

Beispiel 15

2,4-Bis-(tert.butylamino)-6-pentylamino-1,3,5-triazin Fp. 108°C

Beispiel 16

2,4-Bis-(tert.butylamino)-6-(3-pentylamino)-1,3,5-triazin Fp. 125°C

Beispiel 17

2,4-Bis-(tert.butylamino)-6-diisobutylamino-1,3,5-triazin Fp. 81°C

Beispiel 18

2,4-Bis-(tert.butylamino)-6-(N-isopropyl-N-methylamino-1,3,5-triazin
Fp. 199°C

Beispiel 19

a) 2,4-Dichlor-6-diisopropylamino-1,3,5-triazin

Zu einer Lösung von 18,4 g 2,4,6-Trichlor-1,3,5-triazin in 100 ml Tetrahydrofuran werden unter Rühren bei 0°C 22 g Diisopropylamin zugetropft. Dann läßt man bei 0°C noch 30 Minuten nachrühren. Ausgefallenes Diisopropylamin-hydrochlorid wird abgetrennt und der Filterrückstand wird mit Tetrahydrofuran nachgewaschen. Der aus den vereinigten Filtraten gewonnene Rückstand zeigt im Dünnschichtchromatogramm (siehe Beispiel 1, Fließmittel jedoch Essigsäureethylester/Hexan 1:3) Rf=0,77. Der Schmelzpunkt liegt bei 102°C. Ausbeute 24,3 g (97,5 % der Theorie).

b) 2,4-Bis-(tert.butylamino)-6-diisopropylamino-1,3,5-triazin

12,5 g 2,4-Dichlor-6-diisopropylamino-1,3,5-triazin werden mit 200 ml tert.Butylamin bei 150°C 24 Stunden im Druckgefäß umgesetzt. Das überschüssige tert.Butylamin wird mit Hilfe eines Rotationsverdampfers im Vakuum zurückgewonnen. Den Rückstand löst man nun im Zweiphasensystem von 250 ml Chloroform und 250 ml Wasser. Aus der abgetrennten organischen Phase erhält man das gewünschte 2,4-Bis-(tert.butylamino)-6-diisopropylamino-1,3,5-triazin und kristallisiert aus Ethanol um. Farblose Kristalle. Ausbeute 14,7 g (91 % der Theorie). Schmelzpunkt 145°C. Dünnschichtchromatographie (siehe auch Beispiel 1), Fließmittel Essigsäureethylester Rf 0,56, Detektion UV-Lampe 254 nm.

Beispiel 20

2,4-Bis-(tert.butylamino)-6-dimethylamino-1,3,5-triazin Fp. 192°C
Dieses Triazinderivat wird analog Beispiel 19b mit Hilfe von 2,4-Dichlor-6-dimethylamino-1,3,5-triazin (Fp. 108°C) hergestellt.

Beispiel 21

2,4-Bis-(tert.butylamino)-6-(1,1-dimethyl-2-hydroxy-ethyl amino)-1,3,5-triazin
45 g 2-Amino-2-methylpropanol (1) und 12,9 g 2,4-Bis-(tert.butylamino)-6-chlor-1,3,5-triazin werden 3 Stunden bei 150°C gerührt. Das Reaktionsgemisch rührt man anschließend mit 1000 ml destilliertem Wasser bei ca. 35-40°C gut durch. Der unlösliche Anteil wird isoliert und mit Wasser nachgewaschen. Nach dem Trocknen im Vakuum kristallisiert man aus Toluol um. Ausbeute 14,2 g (91,0 % der Theorie). Fp. 163°C. DC siehe Beispiel 1, Fließmittel Essigsäureethylester Rf=0,38.

Beispiel 22

2,4-Bis-(tert.butylamino)-6-(1,1-bis-hydroxymethylethylamino-)-1,3,5-triazin
Fp. 198°C

Beispiel 23

2,4-Bis-(tert.butylamino)-6-(tris-hydroxymethyl-methylamino)-1,3,5-triazin
Fp. 236°C
Die Beispiele 22 und 23 wurden analog Beispiel 21 erhalten. Beim Beispiel 23 wurde die Reaktionstemperatur auf 175-180°C erhöht.

**Ansprüche**

1. 2,4-Bis-(tertiär butylamino)-1,3,5-triazine der allgemeinen Formel I

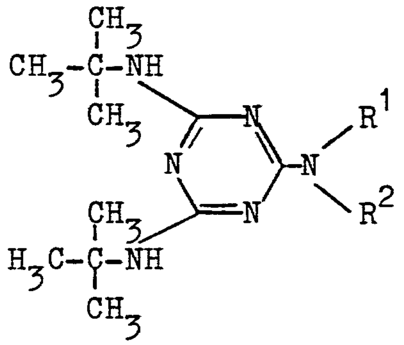

I

worin
$R^1$ $C_1$-$C_6$-Alkyl, $C_2$-$C_6$-Monohydroxyalkyl, $C_3$-$C_6$-Dihydroxyalkyl, $C_4$-$C_6$-Trihydroxyalkyl, Alkoxyalkyl mit insgesamt 3 bis 7 Kohlenstoffatomen, $C_3$-$C_7$-Alkenyl, $C_3$-$C_7$-Cycloalkyl, Cycloalkylalkyl mit insgesamt 4 bis 7 Kohlenstoffatomen, $C_1$-$C_6$-Alkylamino, Dialkylamino mit insgesamt 2 bis 10 Kohlenstoffatomen, oder einen

Amino

rest der Formel 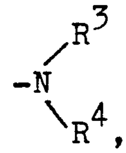 ,

worin

$R^3$ und $R^4$ zusammen eine Alkylenkette mit 3 bis 6 Methylengruppen darstellen, von denen gegebenenfalls eine durch O oder S ersetzt ist oder von denen eine gegebenenfalls mit $C_1$-$C_3$-Alkyl substituiert ist, bedeutet und

$R^2$ Wasserstoff, $C_1$-$C_6$-Alkyl, $C_2$-$C_6$-Hydroxyalkyl, Alkoxyalkyl mit insgesamt 3 bis 7 Kohlenstoffatomen oder $C_3$-$C_7$-Alkenyl ist oder

$R^1$ und $R^2$ bilden gemeinsam mit dem Stickstoffatom ein 4 bis 7 gliedriges, 1, 2 oder 3 Stickstoffatome enthaltendes, ungesättigtes, teilhydriertes oder gesättigtes heterocyclisches Ringsystem, das gegebenenfalls mit $C_1$-$C_3$-Alkyl substituiert ist, oder $R^1$ und $R^2$ bilden gemeinsam mit dem Stickstoffatom den Morpholin oder Thiomorpholinrest,

wobei jedoch nicht gleichzeitig $R^1$ Tertiärbutyl und $R^2$ Wasserstoff bedeuten.

2. Verbindungen gemäß Anspruch 1, dadurch gekennzeichnet, daß in Formel I

$R^1$ $C_1$-$C_6$-Alkyl, $C_4$-$C_6$-Mono-, Di-oder Tri-hydroxyalkyl, $C_5$-$C_6$-Cycloalkyl, Dialkylamino mit insgesamt 2 bis 4

Kohlenstoffatomen, oder einen Aminorest 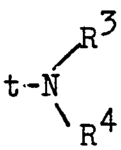 ,worin

$R^3$, $R^4$ und das Stickstoffatom gemeinsam einen Pyrrolidino-, Piperidino, Morpholino-oder Thiomorpholinorest, darstellen bedeutet.

$R^2$ Wasserstoff, $C_1$-$C_6$-Alkyl oder $C_4$-$C_6$-Monohydroxyalkyl ist, oder

$R^1$ und $R^2$ gemeinsam mit dem Stickstoffatom einen heterocyclischen 5-6-gliedrigen Ring bilden, der 1 bis 2 Stickstoffatome aufweist und gesättigt oder ungesättigt ist.

3. Verfahren zur Herstellung von 2,4-Bis-(tertiär butylamino)-1,3,5-triazinen gemäß Anspruch 1, dadurch gekennzeichnet, daß man

a) eine Verbindung der allgemeinen Formel II

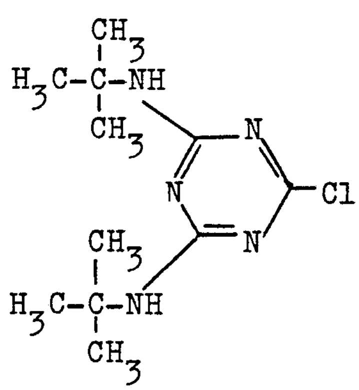

II

und ein Amin der allgemeinen Formel III,

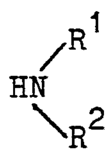

III

worin $R^1$ und $R^2$ die gleichen Bedeutungen wie in der Formel I haben, miteinander zu einer Verbindung der Formel I, worin $R^1$ und $R^2$ die angegebenen Bedeutungen haben, umsetzt oder

b) eine Verbindung der allgemeinen Formel IV,

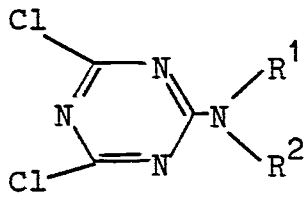

IV

worin $R^1$ und $R^2$ ebenfalls die gleichen Bedeutungen wie in der Formel I haben, mit tertiär Butylamin umsetzt.

4. Verwendung einer Verbindung gemäß Anspruch 1 zur Behandlung epileptischer Erkrankungen und pathologischer Angstzustände.

5. Verfahren zur Behandlung epileptischer Erkrankungen und pathologischer Angstzustände durch Applikation einer wirksamen Menge einer Verbindung gemäß Anspruch 1.

6. Arzneimittel enthaltend eine wirksame Menge einer Verbindung gemäß Anspruch 1.

7. Verfahren zur Herstellung eines Arzneimittels, dadurch gekennzeichnet, daß man eine wirksame Menge einer Verbindung gemäß Anspruch 1 in eine geeignete Darreichungsform überführt.

Patentansprüche für die folgenden Vertragsstaaten: Griechenland, Österreich und Spanien:

1. Verfahren zur Herstellung von 2,4-Bis-(tertiärbutylamino)-1,3,5-triazinen der allgemeinen Formel I

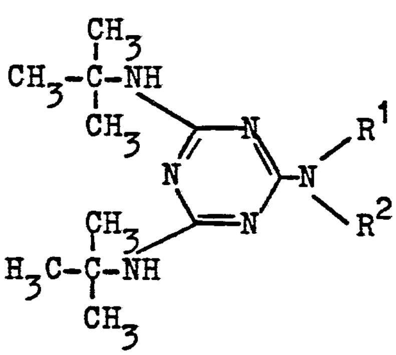

I

worin

$R^1$ $C_1$-$C_6$-Alkyl, $C_2$-$C_6$-Monohydroxyalkyl, $C_3$-$C_6$-Dihydroxyalkyl, $C_4$-$C_6$-Trihydroxyalkyl, Alkoxyalkyl mit insgesamt 3 bis 7 Kohlenstoffatomen, $C_3$-$C_7$-Alkenyl, $C_3$-$C_7$-Cycloalkyl, Cycloalkylalkyl mit insgesamt 4 bis 7 Kohlenstoffatomen, $C_1$-$C_6$-Alkylamino, Dialkylamino mit insgesamt 2 bis 10 Kohlenstoffatomen, oder einen Amino-

rest der Formel 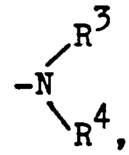 .

worin

$R^3$ und $R^4$ zusammen eine Alkylenkette mit 3 bis 6 Methylengruppen darstellen, von denen gegebenenfalls eine durch O oder S ersetzt ist oder von denen eine gegebenenfalls mit $C_1$-$C_3$-Alkyl substituiert ist, bedeutet und

$R^2$ Wasserstoff, $C_1$-$C_6$-Alkyl, $C_2$-$C_6$-Hydroxyalkyl, Alkoxyalkyl mit insgesamt 3 bis 7 Kohlenstoffatomen oder $C_3$-$C_7$-Alkenyl ist oder

$R^1$ und $R^2$ bilden gemeinsam mit den Stickstoffatom ein 4 bis 7 gliedriges, 1, 2 oder 3 Stickstoffatome enthaltendes, ungesättigtes, teilhydriertes oder gesättigtes heterocyclisches Ringsystem, das gegebenenfalls mit $C_1$-$C_3$-Alkyl substituiert ist, oder $R^1$ und $R^2$ bilden gemeinsam mit dem Stickstoffatom den Morpholin oder Thiomorpholinrest,

wobei jedoch nicht gleichzeitig $R^1$ Tertiärbutyl und $R^2$ Wasserstoff bedeuten, dadurch gekennzeichnet, daß man

a) eine Verbindung der allgemeinen Formel II

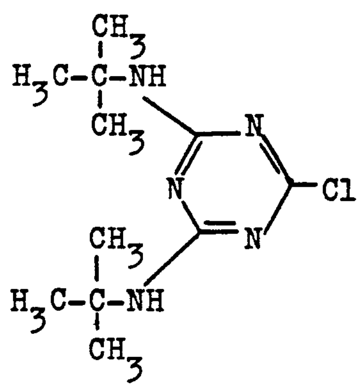

II

und ein Amin der allgemeinen Formel III,

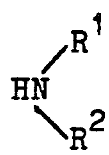

III

worin $R^1$ und $R^2$ die gleichen Bedeutungen wie in der Formel I haben, miteinander zu einer Verbindung der Formel I, worin $R^1$ und $R^2$ die angegebenen Bedeutungen haben, umsetzt oder

b) eine Verbindung der allgemeinen Formel IV,

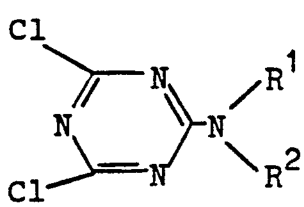

IV

worin $R^1$ und $R^2$ ebenfalls die gleichen Bedeutungen wie in der Formel I haben, mit tertiär Butylamin umsetzt.

2. Verfahren gemäß Anspruch 1, dadurch gekennzeichnet, daß man eine Verbindung der Formel I worin

$R^1$ $C_1$-$C_6$-Alkyl, $C_4$-$C_6$-Mono-, Di-oder Tri-hydroxyalkyl, $C_5$-$C_6$-Cycloalkyl, Dialkylamino mit insgesamt 2 bis 4 Kohlenstoffatomen, oder einen Aminorest-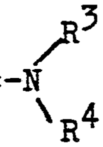,worin

$R^3$, $R^4$ und das Stickstoffatom gemeinsam einen Pyrrolidino-, Piperidino, Morpholino-oder Thiomorpholinorest, darstellen, bedeutet,

$R^2$ Wasserstoff, $C_1$-$C_6$-Alkyl oder $C_4$-$C_6$-Monohydroxyalkyl ist, oder

$R^1$ und $R^2$ gemeinsam mit dem Stickstoffatom einen heterocyclischen 5-6-gliedrigen Ring bilden, der 1 bis 2 Stickstoffatome aufweist und gesättigt oder ungesättigt ist, herstellt.

3. 2,4-Bis-(tertiärbutylamino)-1,3,5-triazine der in Anspruch 1 angegebenen Formel I erhältlich nach Verfahren gemäß Anspruch 1.

4. Verfahren zur Herstellung eines Arzneimittels, dadurch gekennzeichnet, daß man eine wirksame Menge einer Verbindung der Formel I gemäß Anspruch 1 in eine geeignete Darreichungsform überführt.

Europäisches Patentamt

# EUROPÄISCHER RECHERCHENBERICHT

Nummer der Anmeldung

EP 87 10 4465

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 96, Nr. 8, 22. Februar 1982, Seite 708, Spalte 1, Zusammenfassungsnr. 62336e, Columbus, Ohio, US; D.J. SUBACH "Liquid chromatography of the s-triazines", & CHROMATOGRAPHIA 1981, 14(6), 371-373 in Verbindung mit CHEMICAL ABSTRACTS, Band 96, CHEMICAL SUBSTANCE INDEX, PROD-Z, January-June 1982, Seite 6706CS, Spalte 2, Zeilen 18-21, Columbus, Ohio, US; sowie CHEMICAL ABSTRACTS, Band 96, FORMULA INDEX, A-C15, 1982, Seite 1090F, Spalte 2, Zeilen 27, 30-32, Columbus, Ohio, US; --- | 1,2 | C 07 D 251/70<br>C 07 D 403/04<br>A 61 K 31/53 |
| D,A | US-A-4 269 832 (A.S. TOMCUFCIK et al.)<br>* Spalten 1-3, Zeile 34; Beispiel 19 * --- | 1,3 | |
| A | DE-A-2 025 080 (AMERICAN CYANAMID CO.)<br>* Anspruch 42; Beispiel 22 * & US - A - 3 591 693 (Kat. D,A) --- | 1,6 | **RECHERCHIERTE SACHGEBIETE (Int. Cl.4)**<br>C 07 D 251/00<br>C 07 D 403/04 |
| D,A | US-A-2 691 021 (D.W. KAISER et al.)<br>* Anspruch 1 * --- -/- | 1 | |

Der vorliegende Recherchenbericht wurde für alle Patentansprüche erstellt.

| Recherchenort | Abschlußdatum der Recherche | Prüfer |
|---|---|---|
| BERLIN | 07-07-1987 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

**EUROPÄISCHER RECHERCHENBERICHT**

Nummer der Anmeldung

EP 87 10 4465

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | BULLETIN DE LA SOCIETE CHIMIQUE DE FRANCE, Nr. 6, Teil 2, 1973, Seiten 2112-2115; P. CAUBERE et al.: "Condensations d'amines sur quelques chloro-6 diamino-2,4 triazines-1,3,5 en milieu aprotique" * Seite 2112, Schema 1; Seite 2113, Tabelle II * | 3 | |
| A | CHEMICAL ABSTRACTS, Band 101, Nr. 23, 3. Dezember 1984, Seite 648, Spalte 1, Zusammenfassungsnr. 211107g, Columbus, Ohio, US; A. KREUTZBERGER et al.: "Synthesis and trichomonacidal activity of bis(isopropylamino)-1,3,5-triazines", & ARCH. PHARM. (WEINHEIM, GER.) 1984, 317(9), 754-759 | 3 | RECHERCHIERTE SACHGEBIETE (Int. Cl.4) |
| A | EP-A-0 073 974 (BAYER AG) * Anspruch 6 * | 3 | |

Der vorliegende Recherchenbericht wurde für alle Patentansprüche erstellt.

| Recherchenort | Abschlußdatum der Recherche | Prüfer |
|---|---|---|
| BERLIN | 07-07-1987 | HASS C V F |

KATEGORIE DER GENANNTEN DOKUMENTE
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument